# EUROPEAN PATENT SPECIFICATION

(11) **EP 1 775 306 B1**
(45) Date of publication and mention of the grant of the patent: **08.04.2009**
(21) Application number: 05292121.0
(22) Date of filing: 11.10.2005
(51) Int. Cl.: C07K 7/06, A61K 38/08

(54) **ckrox peptides and analogs thereof for treating skin ageing**
ckrox-Peptide und ihre Analoge zur Behandlung von Hautalterung
Peptides de ckrox et leurs analogues pour le traitement du vieillissement cutane

(43) Date of publication of application: 18.04.2007
(73) Proprietor: Johnson & Johnson Consumer France SAS, 92787 Issy les Moulineaux Cedex 9 (FR)
(72) Inventor: Oddos, Thierry, 92190 Meudon (FR); Bellemere, Gaëlle, 76000 Rouen (FR)
(74) Representative: Metten, Karl-Heinz

(56) References cited:
- US-A1- 2005 065 090
- TAMURA T ET AL: "ANTIBODIES AGAINST SYNTHETIC PEPTIDES AS A TOOL FOR FUNCTIONAL ANALYSIS OF THE TRANSFORMING PROTEIN PP-60-SRC" CELL, vol. 34, no. 2, 1983, pages 587-596, XP002367811 ISSN: 0092-8674
- DATABASE EMBL [Online] 1 October 1995 (1995-10-01), "Homo sapiens Ig gene V segment, D segment, and J segment." XP002367817 retrieved from EBI accession no. EM_PRO:HSVDJI Database accession no. L37848
- DATABASE EMBL [Online] 24 November 1993 (1993-11-24), "transthyretin [human, Genomic Mutant, 9 nt]." XP002367818 retrieved from EBI accession no. EM_PRO:S60148 Database accession no. S60148
- DATABASE EMBL [Online] 18 February 2004 (2004-02-18), "Chlamydomonas reinhardtii strain CC-2290 AC206 (AC206) gene, 3' UTR and partial cds." XP002367819 retrieved from EBI accession no. EM_PRO:AY453426 Database accession no. AY453426
- DATABASE EMBL [Online] 1 October 1995 (1995-10-01), "Homo sapiens Ig gene V segment, D segment, and J segment." XP002367820 retrieved from EBI accession no. EM_PRO:HSVDJV Database accession no. L37861
- GALERA PHILIPPE ET AL: "C-Krox, a transcriptional regulator of type I collagen gene expression, is preferentially expressed in skin" PROCEEDINGS OF THE NATIONAL ACADEMY OF SCIENCES OF THE UNITED STATES OF AMERICA, vol. 91, no. 20, 1994, pages 9372-9376, XP002367812 ISSN: 0027-8424

## Description

### FIELD OF THE INVENTION

The present invention relates peptides, compositions containing such peptides, and the use thereof.

### BACKGROUND OF THE INVENTION

C-krox is a transcriptional regulator of type I collagen gene expression that preferentially expressed in skin. It has been shown that c-krox can bind to two GC-rich sequences and an additional site in the promoter of the α1(I) collagen gene and to three sites in the promoter of the α2(I) collagen gene. One of the binding sites present in both promoters is adjacent to the CCAAT box. Thus, c-krox is one transcription factor controlling the coordinated expression of the two type I collagen genes in skin (P. Galera et al., Proc. Natl. Acad. Sci. Vol. 91, 9372-76, 1994). In addition, C-Krox is also known to play a major role in type II collagen expression and the chondrocyte phenotype modulation in human cells (C. Ghayor et al., J. of Biol. Chem., Vol. 275, No. 35, 27421-38, 2000).

C-Krox is a protein that displays a negative regulating effect on the expression of collagen in human dermal fibroblasts (B. Peterkofsky et al., J. of Cellular Biochem. Vol. 73, 408-22, 1999). C-krox is present predominantly in skin and its expression is higher in adult tissue than in newborn tissue (C. Ghayor et al., 2000). Thus, inhibition of the function of this protein should result in the higher synthesis of extracellular matrix through the use of peptide derived from its sequence and especially the one derived from the sequence binding to the DNA.

C-krox typically contains three zinc finger domains. The binding sequences of these domains have been elucidated. Applicants have derived peptides from binding sequences that stimulate the collagen synthesis of normal human dermal fibroblast.

In US 2005/0065090 A1 selected oligopeptides and derivatives thereof as well as peptide analogs and derivatives thereof are disclosed which are reported to diffuse rapidly and in sufficient concentrations through the cell membrane up to the intracellular site of action. These compounds shall lead to a clearly increased production of collagen and fibronectin, thereby exerting a positive and stimulating effect on the extracellular matrix which influences the mechanical and physiological appearance of the skin. Those oligopeptides and peptide analogs are preferred which comprise the following sequences: -Ser-Ser-Orn-, Lys-Thr-Thr-Orn-Ser, Lys-Thr-Thr-Dab-sir and Lys-Thr-Thr-Dab-Ser.

### SUMMARY OF THE INVENTION

In one aspect, the present invention features an isolated peptide of the formula I 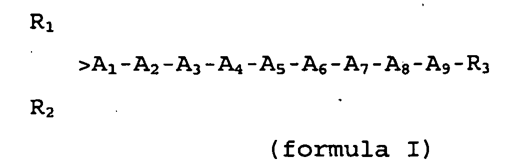 wherein formula has at least six amino acid residues; and:
A₁ is Val;
A₂ is Arg;
A₃ is Phe;
A₄ is Thr;
A₅ is Arg;
A₆ is Asn;
A₇ is Asp, or absent;
A₈ is Lys, or absent;
A₉ is Leu, or absent;
or wherein
A₁ is Val, or absent;
A₂ is Arg, or absent;
A₃ is Phe, or absent;
A₄ is Thr;
A₅ is Arg ;
A₆ is Asn;
A₇ is Asp;
A₈ is Lys;
A₉ is Leu;
A'₉ is Leu, Met, Val, Ile or Phe;
A'₁₀ is Lys or Arg;
A'₁₁ is Asn, Asp, Gly or Gln;
each R'₁ and R'₂, independently, is H, C₁₋₁₂ alkyl, C₇₋₁₀ phenylalkyl, or C(=O)E₁, where E'₁ is C₁₋₁₂ alkyl, C₃₋₁₄ alkenyl, C₃₋₁₄ alkynyl, phenyl, 3,4-dihydroxyphenylalkyl, naphthyl, or C₇₋₁₀ phenylalkyl; provided that when either R₁ or R₂ is C(=O)E'₁, the other must be H; and
R'₃ is OH, NH₂, C₁₋₁₂ alkoxy, C₇₋₁₀ phenylalkoxy, C₁₁₋₁₄ naphthylalkoxy, C₁₋₁₂ alkylamino, C₇₋₁₀ phenylalkylamino, or C₁₁₋₁₄ naphthylalkylamino;
or a cosmetically-acceptable salt thereof;
(b) a peptide of Formula III, 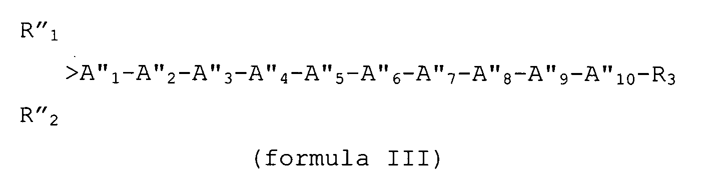 wherein:
A"₁ is Cys or Ser;
A"₂ is His, Tyr or Phe;
A"₃ is Lys or Arg;
A"₄ is Leu, Met, Val, Ile or Phe;
A"₅ is Leu, Met, Val, Ile or Phe;
A"₆ is His, Tyr or Phe;
A"₇ is Asn, Asp, Gly or Gln;
A"₈ is Val, Ala, Leu or Met;
A"₉ is Asn, Asp, Gly or Gln;
A"₁₀ is Lys or Arg;

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Unless otherwise indicated, a percentage refers to a percentage by weight (i.e., %(W/W)).

### Definitions

What is meant by a "product" is a product in finished packaged form. In one embodiment, the package is a container such as a plastic, metal or glass tube or jar containing the composition. The product may further contain additional packaging such as a plastic or cardboard box for storing such container.

In one embodiment, the product contains instructions directing the user to administer the composition to the skin to treat at least one sign of aging on the skin selected from the group consisting of enhancing the elasticity of the skin, enhancing the firmness of the skin, smoothing the surface of the skin, and reducing the appearance of wrinkles on the skin.

What is meant by "enhancing the elasticity" of the skin is enhancing the elasticity or preventing the loss of elasticity of the skin.

What is meant by "enhancing the firmness" of the skin is enhancing the firmness or preventing the loss of firmness of the skin.

As used herein, the term "wrinkle" includes fine line, fine wrinkles, coarse wrinkles, cellulite, scars, and stretch marks. Examples of wrinkles include, but are not limited to, fine lines around the eyes (e.g., "crow's feet"), forehead and cheek wrinkles, frown-lines, and laugh-lines around the mouth.

What is meant by "promoting" is promoting, advertising, or marketing. Examples of promoting include, but are not limited to, written, visual, or verbal statements made on the product or in stores, magazines, newspaper, radio, television, internet, and the like.

For promoting the treatment of signs of aging, examples of such statements include, but are not limited to, "enhances skin elasticity," "helps reduce the appearance of photodamaged skin," "improving visible and tactilely perceptible manifestations of the skin," "increases skin elasticity or firmness," "restores skin elasticity," "treats sagging or lax skin," "reduces the appearance of cellulite," "lifts the skin," "younger looking skin," "smoothing under eye bags," "smoothing skin texture," "lifts the face," "younger skin," and "makes skin look younger."

As used herein, "administering to skin in need of such treatment" means contacting (e.g., by use of the hands or an applicator such, but not limited to, a wipe, tube, roller, spray, or patch) the area of skin in need such treatment. These features may be present on the face such as under or adjacent the eyes, or on the forehead, cheeks, jowls, and neck as well as other areas of the body such as the arms and legs (e.g., cellulite).

As used herein, "composition" means a composition suitable for administration to the skin.

As used herein, "cosmetically-acceptable" means that the ingredients or compositions which the term describes are suitable for use in contact with the skin without undue toxicity, incompatibility, instability, irritation, allergic response, and the like. This term is not intended to limit the ingredient/composition to which it describes for use solely as a cosmetic (e.g., the ingredient/composition may be a pharmaceutical agent).

As used herein, "safe and effective amount" means an amount of the compound, carrier, or of the composition sufficient to induce an enhancement in tissue elasticity, but low enough to avoid serious side effects. The safe and effective amount of the compounds or composition will vary with the area being treated, the age, health and skin type of the end user, the duration and nature of the treatment, the specific compound or composition employed, the particular cosmetically-acceptable carrier utilized, and like factors.

### Peptides

The composition of the present invention contains a peptide of the formula I 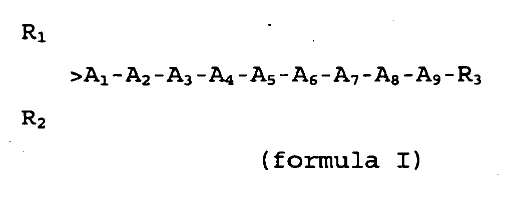 wherein formula I has at least six amino acid residues; and:
A₁ is Val;
A₂ is Arg ;
A₃ is Phe ;
A₄ is Thr ;
A₅ is Arg ;
A₆ is Asn;
A₇ is Asp, or absent;
A₈ is Lys, or absent;
A₉ is Leu, or absent;
or wherein
A₁ is Val, or absent;
A₂ is Arg, or absent;
A₃ is Phe, or absent;
A₄ is Thr;
A₅ is Arg;
A₆ is Asn;
A₇ is Asp;
A₈ is Lys;
A₉ is Leu;
each R₁ and R₂, independently, is H, C₁₋₁₂ alkyl, C₇₋₁₀ phenylalkyl, or C (=O) E₁, where E₁ is C₁₋₁₂ alkyl, C₃₋₁₄ alkenyl, C₃₋₁₄ alkynyl, phenyl, 3,4-dihydroxyphenylalkyl, naphthyl, or C₇₋₁₀ phenylalkyl; provided that when either R₁ or R₂ is C (=O) E₁, the other must be H; and
R₃ is OH, NH₂, C₁₋₁₂ alkoxy, C₇₋₁₀ phenylalkoxy, C₁₁₋₁₄ naphthylalkoxy, C₁₋₁₂ alkylamino, C₇₋₁₀ phenylalkylamino, or C₁₁₋₁₄ naphthylalkylamino;
or a cosmetically acceptable salt thereof.

In one embodiment, R₁ and R₂, which are bound to the N-terminus of the peptide, are both H. In another embodiment, R₁ is H and R₂ is C (=O)E₁ (e.g., palmitoyl, oleatoyl, or stearatoyl).

Examples of peptides of the present invention include, but are not limited to H₂-Val-Arg-Phe-Thr-Arg-Asn-Asp-Lys-Leu-NH₂, H₂-Val-Arg-Phe-Thr-Arg-Asn-Asp-Lys-Leu-OH, H₂-Val-Arg-Phe-Thr-Arg- Asn-NH₂, H₂-Val-Arg-Phe-Thr-Arg-Asn-OH ("Peptide 1"), H₂ -Thr-Arg-Asn-Asp- Lys -Leu-NH₂, H₂-Thr-Arg-Asn-Asp-Lys-Leu-OH ("Peptide 2"), Palmitoyl-Val-Arg-Phe-Thr-Arg-Asn-Asp-Lys-Leu-NH₂, Palmitoyl-Val-Arg-Phe-Thr-Arg-Asn-Asp-Lys-Leu-OH, Palmitoyl-Val-Arg-Phe-Thr-Arg-Asn-NH₂, Palmitoyl-Val-Arg-Phe-Thr-Arg-Asn-OH, Palmitoyl-Thr-Arg-Asn-Asp-Lys-Leu-NH₂, Palmitoyl-Thr-Arg-Asn-Asp-Lys-Leu-OH, Stearatoyl-Val-Arg-Phe-Thr-Arg-Asn-Asp-Lys-Leu-NH₂, Stearatoyl-Val-Arg-Phe-Thr-Arg-Asn-Asp-Lys-Leu-OH, Stearatoyl-Val-Arg-Phe-Thr-Arg-Asn-NH₂, Stearatoyl-Val-Arg-Phe-Thr-Arg-Asn-OH, Stearatoyl-Thr-Arg-Asn-Asp-Lys-Leu-NH₂, Stearatoyl-Thr-Arg-Asn-Asp-Lys-Leu-OH, or a cosmetically-acceptable salt thereof.

The symbol A₁, A₂, or the like used herein (e.g., in Figure 1) stands for the residue of an alpha-amino acid. Such symbols represent the general structure, -NH-CH(X)-CO- or =N-CH(X)-CO- when it is at the N-terminus or -NH-CH(X)-CO- when it is not at the N- terminus, where X denotes the side chain (or identifying group) of the alpha-amino acid, e.g., X is -CH(CH₃)₂ for Val. Note that the N-terminus is at the left and the C-terminus at the right in accordance with the conventional representation of a polypeptide chain. R₁ and R₂ are both bound to the free nitrogen atom N-terminal amino acid (e.g., A₁ or A₂) and the R₃ is bound to the free carboxyl group of the C-terminal amino acid (e.g., A₅ or A₆).

Furthermore, where the amino acid residue is optically active, it is the L-form configuration that is intended unless the D-form is expressly designated. An alkyl group, if not specified, contains 1-12 carbon atoms.

The peptide of the invention can be provided in the form of cosmetically acceptable salts. Examples of preferred salts are those with therapeutically acceptable organic acids, e.g., acetic, palmitic, oleic, stearic, lactic, maleic, citric, malic, ascorbic, succinic, benzoic, salicylic, methanesulfonic, or pamoic acid, as well as polymeric acids such as tannic acid or carboxymethyl cellulose, and salts with inorganic acids such as the hydrohalic acids (e.g., hydrochloric acid), sulfuric acid or phosphoric acid.

The amount of peptide present in the composition will depend on the peptide used. The peptide typically will be present in the composition in an amount from about 0.001% to about 10% by weight, in particular in an amount from about 0.01% to about 1% by weight.

The method for synthesizing peptides of the present invention are well documented and are within the ability of a person of ordinary skill in the art. See, e.g., Bodanszky M, Int J Pept Protein Res 25(5):449-74 (1985), Fmoc Solid Phase Peptide Synthesis, eds. Chan, W. & White, P. (Oxford University Press, 2000), and Chemical Approaches to the Synthesis of Peptides and Proteins, Lloyd-Williams, P. et al. (CRC Press, 1997).

### Compositions

The compositions useful in the present invention involve formulations suitable for administering to the target tissues. In one embodiment, the composition contains a safe and effective amount of (i) the peptide and (ii) a cosmetically-acceptable carrier. In one embodiment, the cosmetically-acceptable carrier is from about 90% to about 99.99%, by weight, of the composition (e.g., from about 95% to about 99%, by weight, of the composition).

The compositions may be made into a wide variety of product types that include but are not limited to solutions, suspensions, lotions, creams, gels, sticks, sprays, ointments, cleansing liquid washes and solid bars, shampoos and hair conditioners, pastes, foams, powders, mousses, shaving creams, wipes, patches, nail lacquers, wound dressing and adhesive bandages, hydrogels, film-forming products, facial and skin masks, make-up such as foundations, mascaras, and lipsticks, liquid drops, vaginal washes, suppositories, tampons, toothpastes, mouthwashes, lozenges, tablets, gums and candy, mucoadhesives, and the like. These product types may contain several types of cosmetically- acceptable carriers including, but not limited to solutions, suspensions, emulsions such as microemulsions and nanoemulsions, gels, solids and liposomes. The following are non-limitative examples of such carriers. Other carriers can be formulated by those of ordinary skill in the art.

The compositions useful in the present invention can be formulated as solutions. Solutions typically include an aqueous or organic solvent (e.g., from about 90% to about 99% of a cosmetically acceptable aqueous or organic solvent). Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, and mixtures thereof.

A lotion can be made from such a solution. Lotions typically contain from about 1% to about 20% (e.g., from about 5% to about 10%) of an emollient(s) and from about 70% to about 90% (e.g., from about 75% to about 80%) of water. As used herein, "emollients" refer to materials used for the prevention or relief of dryness, as well as for the protection of the skin or hair. Examples of emollients include, but are not limited to, those set forth in the International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "ICI Handbook").

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may contain a simple base of animal, vegetable, or synthetic oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 1693-1697.

The compositions useful in the present invention can also be formulated as emulsions. If the carrier is an emulsion, from about 1% to about 10% (e.g., from about 2% to about 5%) of the carrier contains an emulsifier(s). Emulsifiers may be nonionic, anionic or cationic. Examples of emulsifiers include, but are not limited to, those set forth in the ICI Handbook, pp.1673-1686.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (e.g., a wax-based stick, soap bar composition, powder, wipe containing powder, lozenge, suppository, candy, or gum).

The compositions useful in the subject invention may contain, in addition to the aforementioned components, a wide variety of additional oil-soluble materials and/or water-soluble materials conventionally used in compositions for use on skin and mucosal tissues at their art-established levels.

### Additional Cosmetically Active Agents

In one embodiment, the composition further contains another cosmetically active agent in addition to the peptide. What is meant by a "cosmetically active agent" is a compound (e.g., a synthetic compound or a compound isolated from a natural source, or a natural extract containing a mixture of compounds) that has a cosmetic or therapeutic effect on the tissue, including, but not limiting to, lightening agents, darkening agents such as self-tanning agents, anti-acne agents, shine control agents, anti-microbial agents such as anti-yeast agents, anti-fungal, and anti-bacterial agents, antiinflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, anti-callous agents, agents for skin conditioning, anti-cellulite agents, fluorides, teeth whitening agents, anti-plaque agents, and plaque-dissolving agents, and odor-control agents such as odor masking or pH-changing agents.

In one embodiment, the agent is selected from, but not limited to, the group consisting of hydroxy acids, benzoyl peroxide, D-panthenol, octyl methoxycinnimate, titanium dioxide, octyl salicylate, homosalate, avobenzone, carotenoids, free radical scavengers, spin traps, retinoids and retinoid precursors such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, amino acids such a proline, vitamins, lactobionic acid, acetyl-coenzyme A, niacin, riboflavin, thiamin, ribose, electron transporters such as NADH and FADH2, and other botanical extracts such as aloe vera, Feverfew, and Soy, and derivatives and mixtures thereof. The cosmetically active agent will typically be present in the composition of the invention in an amount of from about 0.001% to about 20% by weight of the composition, e.g., about 0.005% to about 10% such as about 0.01% to about 5%.

Examples of vitamins include, but are not limited to, vitamin A, vitamin Bs such as vitamin B3, vitamin B5, and vitamin B12, vitamin C, vitamin K, vitamin E such as alpha, gamma or delta-tocopherol, and derivatives and mixtures thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), different types of tocopherols (e.g., alpha-, gamma-, and delta-tocopherols and their esters such as acetate) and their mixtures, tocotrienols, and ubiquinone. Natural extracts containing antioxidants suitable for use in the compositions of this invention, include, but not limited to, extracts containing flavonoids, isoflavonoids, and their derivatives such as genistein and diadzein (e.g., such as Soy and Clover extracts, extracts containing resveratrol and the like. Examples of such natural extracts include grape seed, green tea, pine bark, and propolis.

### Other Materials

Various other materials may also be present in the compositions useful in the subject invention. These include humectants, proteins and polypeptides, preservatives and an alkaline agent. Examples of such agents are disclosed in the ICI Handbook, pp. 1650-1667. The compositions of the present invention may also contain chelating agents (e.g., EDTA) and preservatives (e.g., parabens). Examples of suitable preservatives and chelating agents are listed in pp. 1626 and 1654-55 of the ICI Handbook. In addition, the compositions useful herein can contain conventional cosmetic adjuvants, such as colorants such as dyes and pigments, opacifiers (e.g., titanium dioxide), and fragrances.

### Mineral Water

The compositions of the present invention may be prepared using a mineral water, for example mineral water that has been naturally mineralized such as Evian® Mineral Water (Evian, France). In one embodiment, the mineral water has a mineralization of at least about 200 mg/L (e.g., from about 300 mg/L to about 1000 mg/L). In one embodiment, the mineral water contains at least about 10 mg/L of calcium and/or at least about 5 mg/L of magnesium.

The composition and formulations containing such compositions of the present invention may be prepared using methodology that is well known by an artisan of ordinary skill.

### Use

The peptides and compositions of the present invention may be topically applied to the skin. In one embodiment, the invention features a method of treating at least one sign of aging on the skin selected from the group consisting of enhancing the elasticity of the skin, enhancing the firmness of the skin, smoothing the surface of the skin, and reducing the appearance of wrinkles on the skin wherein the method includes applying to skin in need of such treatment at least one peptide of formula I, II, or III or composition containing such peptide(s).

### Example 1

The stimulation of collagen synthesis by peptides of the present invention was evaluated by measuring the incorporation of tritiated proline in fibroblasts collagenic fractions. The assay was conducted as follows. Human fibroblasts (obtained from the human biopsy of a 42 year old) were seeded in 24-well plates at approximately 40,000 cells per well in 1 ml of culture medium. The culture medium contained DMEM (Invitrogen S.a.r.l., Cergy Pontoise, France) supplemented with 10% Fetal Calf Serum (FCS, Cambrex Bio Science Paris S.a.r.l., Emerainville, France), 1% Glutamax (Invitrogen S.a.r.l.), 4 µg/ml gentamicine (Invitrogen S.a.r.l.), and 2.5 µg/ml Fungizone (Invitrogen S.a.r.l.). Cells were placed at 37°C under 5% CO₂ in a humidified atmosphere for 72 hours.

The culture medium was the removed and replaced with 1 ml of either: (i) incubation medium alone (untreated control); (ii) incubation medium further containing 1 ng/ml TGFb1 (R&D Systems Europe, Lille, France) for use as a stimulated positive control; or (iii) incubation medium further containing one of the tested peptides at a concentration of 1 µg/ml or 10 µg/ml. The incubation medium contained DMEM supplemented with 2 % FCS, 1% Glutamax, 4 µg/ml gentamicine, 2.5 µg/ml Fungizone, 50 µg/ml ascorbic acid, 50 µg/ml b-aminoproionitrile, and 3.4 µg/ml a ketoglutarate.

During the last 24 hours of incubation, five µCi of tritiated proline (L-2,3-3H proline, Amersham Biosciences Europe, Saclay, France) was also added to each well. Each condition (untreated control, positive control and tested peptide) was performed in triplicate. In order to normalize the results, the same experiment was performed in parallel, except that tritiated proline was omitted. At the end of the treatment, cells were counted with a coulter counter. After 72 hours of treatment, incubation medium (supernatants) were collected in 2 ml microtubes and frozen at -20°C.

In order to perform the collagen extraction, 1 ml of cold 20% TCA (Sigma-Aldrich, Lyon, France) was added to 1 ml of supernatant. The samples were then vortexed, incubated for 5 minutes (min) at -20°C, and let stand on ice for 30 min. The samples were then centrifuged (2300g, 5 min, 4°C). The supernatants were then eliminated, and the pellets were rinsed with 1 ml of cold 5% TCA, vortexed, and centrifuged (2300g, 5 min, 4°C). The pellets were then rinsed twice with 5% TCA following the same procedure as described above. The pellets were then dissolved in 0.3 ml of 0.05 N NaOH and neutralized with 0.1N HCl to reach pH 6-8. Then, 0.2 ml of protein solution was incubated for 2 hours at 37°C in 2x Tris HCl Buffer containing 10 units of collagenase activity (Clostridium hystolyticum Type III collagenase, Sigma-Aldrich, Lyon, France).

The enzymatic reaction was then stopped by precipitation with 500 µl of 15% TCA and 500 µl of 0.75% tannic acid. Samples were then incubated 30 min on ice. The precipitates were then centrifuged (2300g, 5 min, 4°C). The supernatants containing collagen fractions were collected, and scintillation liquid (Ecolume ICN) was added to each sample (5 ml to 100 µl sample). Then, radioactivity was measured in a β-scintillation counter.

The rate of collagen synthesis was calculated as follows: Synthesis rate = (number of dpm/cell number)*1000

The percentage of stimulation was calculated as follows: % of stimulation = [(synthesis rate in sample-synthesis rate in control)/(synthesis rate in control)]*100.

The results are depicted in Table 1.

**Table 1**

| **Incubation Medium** | **Percent stimulation vs. control** |
|---|---|
| TGF b (1 ng/ml) | 45.7 |
| Peptide 1 (1 ug/ml) | 14.8 |
| Peptide 1 (10 ug/ml) | 43.1 |
| Peptide 2 (1 ug/ml) | 23.5 |
| Peptide 2 (10 ug/ml) | 42.6 |

The results show that the peptides VFTRN and TRNDKL were able to markedly stimulate collagen synthesis at 10 µg/ml for both peptides by 43% and 42% respectively, a level of stimulation similar to the one of TGFb (+45%) a potent stimulator of collagen synthesis in dermal fibroblast used as positive control. At lower concentration (1µg/ml) both peptide stimulate also collagen synthesis but to a lower extent (+15% and + 25% respectively).

## Claims

1. An isolated peptide of the formula I 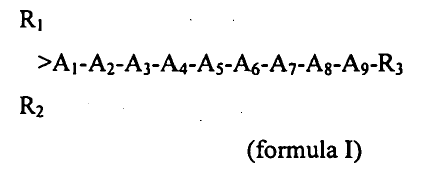 wherein formula I has at least six amino acid residues; and:
A₁ is Val;
A₂ is Arg;
A₃ is Phe;
A₄ is Thr;
A₅ is Arg;
A₆ is Asn;
A₇ is Asp, or absent;
A₈ is Lys, or absent;
A₉ is Leu, or absent;
or wherein
A₁ is Val, or absent;
A₂ is Arg, or absent;
A₃ is Phe, or absent;
A₄ is Thr;
A₅ is Arg;
A₆ is Asn;
A₇ is Asp;
A₈ is Lys;
A₉ is Leu;
each R₁ and R₂, independently, is H, C₁₋₁₂ alkyl, C₇₋₁₀ phenylalkyl, or C(=O)E₁, where E, is C₁₋₁₂ alkyl, C₃₋₁₄ alkenyl, C₃₋₁₄ alkynyl, phenyl, 3,4-dihydroxyphenylalkyl, naphthyl, or C₇₋₁₀ phenylalkyl; provided that when either R₁ or R₂ is C(=O)E₁, the other must be H; and
R₃ is OH, NH₂, C₁₋₁₂ alkoxy, C₇₋₁₀ phenylalkoxy, C₁₁₋₁₄ naphthylalkoxy, C₁₋₁₂ alkylamino, C₇₋₁₀ phenylalkylamino, or C₁₁₋₁₄ naphthylalkylamino;
or a cosmetically acceptable salt thereof.

2. An isolated peptide of claim 1, wherein R₁ is H, R₂ is H or C(=O)E₁ where E₁ is C₁₋₁₂ alkyl, and R₃ is OH or NH₂.

3. An isolated peptide of claim 1, wherein A₁ is Val; A₂ is Arg; A₃ is Phe; A₄ is Thr; A₅ is Arg; A₆ is Asn; A₇ is absent; A₈ is absent; and A₉ is absent.

4. An isolated peptide of claim 1, wherein A₁ is absent; A₂ is absent; A₃ is absent; A₄ is Thr; A₅ is Arg; A₆ is Asn; A₇ is Asp; A₈ is Lys; and A₉ is Leu.

5. An isolated peptide of claim 1, wherein A₁ is Val; A₂ is Arg; A₃ is Phe; A₄ is Thr; A₅ is Arg; A₆ is Asn; A₇ is Asp; A₈ is Lys; and A₉ is Leu.

6. An isolated peptide of any of the preceding claims, wherein R₁ is H, R₂ is H or C(=O)E₁ where E₁ is C₁₋₁₂ alkyl, and R₃ is OH or NH₂.

7. An isolated peptide of any of the preceding claims, wherein R₁ is H, R₂ is H or C(=O)E₁ where E₁ is C₁₋₂₀ alkyl, and R₃ is OH or NH₂.

8. An isolated peptide of claim 1, wherein said peptide is H₂-Val-Arg-Phe-Thr-Arg-Asn-Asp-Lys-Leu-NH₂, H₂-Val-Arg-Phe-Thr-Arg-Asn-Asp-Lys-Leu-OH, H₂-Val-Arg-Phe-Thr-Arg-Asn -NH₂, H₂-Val-Arg-Phe-Thr-Arg-Asn-OH, H₂-Thr-Arg-Asn-Asp-Lys-Leu-NH₂, H₂-Thr-Arg-Asn-Asp-Lys-Leu-OH, Palmitoyl-Val-Arg-Phe-Thr-Arg-Asn-Asp-Lys-Leu-NH₂, Palmitoyl-Val-Arg-Phe-Thr-Arg-Asn-Asp-Lys-Leu-OH, Palmitoyl-Val-Arg-Phe-Thr-Arg-Asn - NH₂, Palmitoyl-Val-Arg-Phe-Thr-Arg-Asn-OH, Palmitoyl-Thr-Arg-Asn-Asp-Lys-Leu-NH₂, Palmitoyl-Thr-Arg-Asn-Asp-Lys-Leu-OH, Stearatoyl-Val-Arg-Phe-Thr-Arg-Asn-Asp-Lys-Leu-NH₂, Stearatoyl-Val-Arg-Phe-Thr-Arg-Asn-Asp-Lys-Leu-OH, Stearatoyl-Val-Arg-Phe-Thr-Arg-Asn-NH₂, Stearatoyl-Val-Arg-Phe-Thr-Arg-Asn-OH, Stearatoyl-Thr-Arg-Asn-Asp-Lys-Leu-NH₂, or Stearatoyl-Thr-Arg-Asn-Asp-Lys-Leu-OH, or a cosmetically-acceptable salt thereof.

9. A composition comprising a peptide of any of the preceding claims and a cosmetically-acceptable carrier.

10. Use of an isolated peptide according to any of the preceding claims for the preparation of a cosmetical or pharmaceutical composition for the treatment of the skin, in particular for enhancing the elasticity of the skin, enhancing the firmness of the skin, smoothing the surface of the skin, or reducing the appearance of wrinkles on the skin.

## Patentansprüche

1. Isoliertes Peptid mit der Formel I 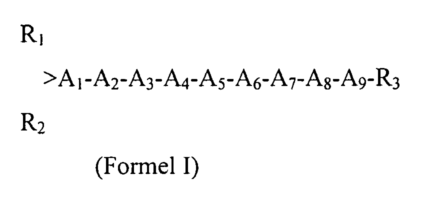 wobei die Formel I mindestens sechs Aminosäurereste aufweist und
A₁ Val ist,
A₂ Arg ist,
A₃ Phe ist,
A₄ Thr ist,
A₅ Arg ist,
A₆ Asn ist,
A₇ Asp ist oder nicht vorhanden ist,
A₈ Lys ist oder nicht vorhanden ist,
A₉ Leu ist oder nicht vorhanden ist
oder wobei
A₁ Val ist oder nicht vorhanden ist,
A₂ Arg ist oder nicht vorhanden ist,
A₃ Phe ist oder nicht vorhanden ist,
A₄ Thr ist,
A₅ Arg ist,
A₆ Asn ist,
A₇ Asp ist,
A₈ Lys ist,
A₉ Leu ist,
wobei R₁ und R₂ unabhängig jeweils H, C₁₋₂-Alkyl, C₇₋₁₀-Phenylalkyl oder C(=O)E₁ ist, wobei E₁ C₁₋₁₂-Alkyl, C₃₋₁₄-Alkenyl, C₃₋₁₄-Alkynyl, Phenyl, 3,4-Dihydroxyphenylalkyl, Naphthyl oder C₇₋₁₀-Phenylalkyl ist, mit der Maßgabe, dass, wenn entweder R₁ oder R₂ C(=O)E₁ ist, das andere H sein muss, und
R₃ OH, NH₂, C₁₋₁₂-Alkoxy, C₇₋₁₀-Phenylalkoxy, C₁₁₋₁₄-Naphthylalkoxy, C₁₋₁₂-Alkylamino, C₇₋₁₀-Phenylalkylamino oder C₁₁₋₁₄-Naphthylalkylamino ist,
oder ein kosmetisch akzeptables Salz davon.

2. Isoliertes Peptid nach Anspruch 1, **dadurch gekennzeichnet, dass** R₁ H ist, R₂ H oder C(=O)E₁ ist, wobei E₁ C₁₋₁₂-Alkyl ist, und R₃ OH oder NH₂ ist.

3. Isoliertes Peptid nach Anspruch 1, **dadurch gekennzeichnet, dass** A₁ Val ist, A₂ Arg ist, A₃ Phe ist, A₄ Thr ist, A₅ Arg ist, A₆ Asn ist, A₇ nicht vorhanden ist, A₈ nicht vorhanden ist und A₉ nicht vorhanden ist.

4. Isoliertes Peptid nach Anspruch 1, **dadurch gekennzeichnet, dass** A₁ nicht vorhanden ist, A₂ nicht vorhanden ist, A₃ nicht vorhanden ist, A₄ Thr ist, A₅ Arg ist, A₆ Asn ist, A₇ Asp ist, A₈ Lys ist und A₉ Leu ist.

5. Isoliertes Peptid nach Anspruch 1, **dadurch gekennzeichnet, dass** A₁ Val ist, A₂ Arg ist, A₃ Phe ist, A₄ Thr ist, A₅ Arg ist, A₆ Asn ist, A₇ Asp ist, A₈ Lys ist und A₉ Leu ist.

6. Isoliertes Peptid nach einem der vorangehenden Ansprüche, **dadurch gekennzeichnet, dass** R₁ H ist, R₂ H oder C(=O)E₁ ist, wobei E₁ C₁₋₁₂-Alkyl ist, und R₃ OH oder NH₂ ist.

7. Isoliertes Peptid nach einem der vorangehenden Ansprüche, **dadurch gekennzeichnet, dass** R₁ H ist, R₂ H oder C(=O)E₁ ist, wobei E₁ C₁₋₂₀-Alkyl ist, und R₃ OH oder NH₂ ist.

8. Isoliertes Peptid nach Anspruch 1, **dadurch gekennzeichnet, dass** das Peptid H₂-Val-Arg-Phe-Thr-Arg-Asn-Asp-Lys-Leu-NH₂, H₂-Val-Arg-Phe-Thr-Arg-Asn-Asp-Lys-Leu-OH, H₂-Val-Arg-Phe-Thr-Arg-Asn-NH₂, H₂-Val-Arg-Phe-Thr-Arg-Asn-OH, H₂-Thr-Arg-Asn-Asp-Lys-Leu-NH₂, H₂-Thr-Arg-Asn-Asp-Lys-Leu-OH, Palmitoyl-Val-Arg-Phe-Thr-Arg-Asn-Asp-Lys-Leu-NH₂, Palmitoyl-Val-Arg-Phe-Thr-Arg-Asn-Asp-Lys-Leu-OH, Palmitoyl-Val-Arg-Phe-Thr-Arg-Asn-NH₂, Palmitoyl-Val-Arg-Phe-Thr-Arg-Asn-OH, Palmitoyl-Thr-Arg-Asn-Asp-Lys-Leu-NH₂, Palmitoyl-Thr-Arg-Asn-Asp-Lys-Leu-OH, Stearatoyl-Val-Arg-Phe-Thr-Arg-Asn-Asp-Lys-Leu-NH₂, Stearatoyl-Val-Arg-Phe-Thr-Arg-Asn-Asp-Lys-Leu-OH, Stearatoyl-Val-Arg-Phe-Thr-Arg-Asn-NH₂, Stearatoyl-Val-Arg-Phe-Thr-Arg-Asn-OH, Stearatoyl-Thr-Arg-Asn-Asp-Lys-Leu-NH₂ oder Stearatoyl-Thr-Arg-Asn-Asp-Lys-Leu-OH oder ein kosmetisch akzeptables Salz davon ist.

9. Zusammensetzung, die ein Peptid nach einem der vorangehenden Ansprüche und einen kosmetisch akzeptablen Träger umfasst.

10. Verwendung eines isolierten Peptids nach einem der vorangehenden Ansprüche zur Herstellung einer kosmetischen oder pharmazeutischen Zusammensetzung zur Behandlung der Haut, insbesondere zur Erhöhung der Elastizität der Haut, Erhöhung der Straffheit der Haut, Glättung der Oberfläche der Haut oder Verminderung des Auftretens von Falten auf der Haut.

## Revendications

1. Peptide isolé selon la formule 1 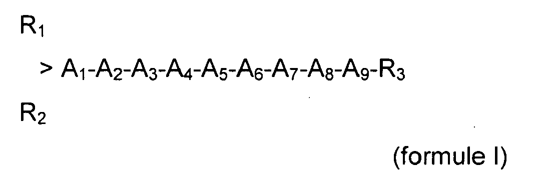 dans lequel la formule 1 présente au moins six résidus d'acides aminés ; et :
A₁ est Val ;
A₂ est Arg ;
A₃ est Phe ;
A₄ est Thr ;
A₅ est Arg ;
A₆ est Asn ;
A₇ est Asp, ou absent ;
A₈ est Lys, ou absent ;
A₉ est Leu, ou absent ;
ou dans lequel
A₁ est Val, ou absent ;
A₂ est Arg, ou absent ;
A₃ est Phe, ou absent ;
A₄ est Thr ;
A₅ est Arg ;
A₆ est Asn ;
A₇ est Asp ;
A₈ est Lys ;
A₉ est Leu ;
chaque R₁ et R₂ est indépendamment H, alkyle en C₁₋₁₂, phénylalkyle en C₇-10, ou C(=O)E₁, où E₁ est un alkyle en C₁₋₁₂, un alcényle en C₃₋₁₄, un alcynyle en C3-14, un phényle, un 3,4-dihydroxyphénylalkyle, un naphtyle, ou un phénylalkyle en C₇₋₁₀ ; à condition que lorsque R₁ ou R₂ est C(=O)E₁, l'autre soit H ; et
R₃ est OH, NH₂, un alcoxy en C₁₋₁₂, un phénylalcoxy en C₇₋₁₀, un naphtylalcoxy en C₁₁₋₁₄, un alkylamino en C₁₋₁₂, un phénylalkylamino en C₇₋₁₀, ou un naphtylalkylamino en C₁₁₋₁₄ ;
ou un sel cosmétiquement acceptable de ceux-ci.

2. Peptide isolé selon la revendication 1, dans lequel R₁ est H, R₂ est H ou C(=O)E₁ où E₁ est un alkyle en C₁₋₁₂ et R₃ est OH ou NH₂.

3. Peptide isolé selon la revendication 1, dans lequel A₁ est Val ; A₂ est Arg ; A₃ est Phe ; A₄ est Phr, A₅ est Arg ; A₆ est Asn ; A₇ est absent ; A₈ est absent, et A₉ est absent.

4. Peptide isolé selon la revendication 1, dans lequel A₁ est absent ; A₂ est absent ; A₃ est absent ; A₄ est Thr ; A₅ est Arg ; A₆ est Asn ; A₇ est Asp ; A₈ est Lys ; et A₉ est Leu.

5. Peptide isolé selon la revendication 1, dans lequel A₁ est Val ; A₂ est Arg ; A₃ est Phe ; A₄ est Thr ; A₅ est Arg ; A₆ est Asn ; A₇ est Asp ; A₈ est Lys ; et A₉ est Leu.

6. Peptide isolé selon l'une quelconque des revendications précédentes, dans lequel R₁ est H, R₂ est H ou C(=O)E₁, où E₁ est un alkyle en C₁₋₁₂, et R₃ est OH ou NH₂.

7. Peptide isolé selon l'une quelconque des revendications précédentes, dans lequel R₁ est H, R₂ est H ou C(=O)E₁, où E₁ est un alkyle en C₁₋₂₀, et R₃ est OH ou NH₂.

8. Peptide isolé selon la revendication 1, dans lequel ledit peptide est H₂-Val-Arg-Phe-Thr-Arg-Asn-Asp-Lys-Leu-NH₂, H₂-Val-Arg-Phe-Thr-Arg-Asn-Asp-Lys-Leu-OH, H₂-Val-Arg-Phe-Thr-Arg-Asn-NH₂, H₂-Val-Arg-Phe-Thr-Arg-Asn-OH, H₂-Thr-Arg-Asn-Asp-Lys-Leu-NH₂, H₂-Thr-Arg-Asn-Asp-Lys-Leu-OH, palmitoyle-Val-Arg-Phe-Thr-Arg-Asn-Asp-Lys-Leu-NH₂, palmitoyle-Val-Arg-Phe-Thr-Arg-Asn-Asp-Lys-Leu-OH, palmitoyle-Val-Arg-Phe-Thr-Arg-Asn-NH₂, palmitoyle-Val-Arg-Phe-Thr-Arg-Asn-OH, palmitoyle-Thr-Arg-Asn-Asp-Lys-Leu-NH₂, palmitoyle-Thr-Arg-Asn-Asp-Lys-Leu-OH, stéaroyle-Val-Arg-Phe-Thr-Arg-Asn-Asp-Lys-Leu-NH₂, stéaroyle-Val-Arg-Phe-Thr-Arg-Asn-Asp-Lys-Leu-OH, stéaroyle-Val-Arg-Phe-Thr-Arg-Asn-NH₂, stéaroyle-Val-Arg-Phe-Thr-Arg-Asn-OH, stéaroyle-Thr-Arg-Asn-Asp-Lys-Leu-NH₂, ou stéaroyle-Thr-Arg-Asn-Asp-Lys-Leu-OH, ou un sel cosmétiquement acceptable de ceux-ci.

9. Composition comportant un peptide selon l'une quelconque des revendications précédentes, et un support cosmétiquement acceptable.

10. Utilisation d'un peptide isolé selon l'une quelconque des revendications précédentes, pour la préparation d'une composition cosmétique ou pharmaceutique de traitement cutané, en particulier pour améliorer l'élasticité de la peau, améliorer la fermeté de la peau, adoucir la surface de la peau, ou réduire l'apparence de rides sur la peau.
